# FASCICULE DE BREVET EUROPEEN

(11) **EP 0 861 633 B1**
(45) Date de publication et mention de la délivrance du brevet: **05.01.2005**
(21) Numéro de dépôt: 98400415.0
(22) Date de dépôt: 20.02.1998
(51) Int. Cl.: A61B 17/04

(54) **Dispositif chirurgical d'ancrage osseux, et ancillaire pour sa mise en place**
Knochenankervorrichtung sowie Setzgerät
Bone anchoring device and insertion instrument

(30) Priorité: 28.02.1997 FR 9702422
(43) Date de publication de la demande: 02.09.1998
(73) Titulaire: Fumex, Laurent, 78730 Saint-Arnoult-en-Yvelines (FR)
(72) Inventeur: Fumex, Laurent, 78730 Saint-Arnoult-en-Yvelines (FR)
(74) Mandataire: L'Helgoualch, Jean

(56) Documents cités:
- WO-A-95/32669
- US-A- 5 443 482

## Description

La présente invention concerne un dispositif chirurgical d'ancrage osseux, et plus particulièrement un dispositif chirurgical permettant de réaliser un ancrage de fil de suture ou de fil chirurgical sur un support osseux, notamment en chirurgie orthopédique, traumatologique, gynécologique et carcinologique.

La rupture de tendons ou de ligaments est un accident qui peut survenir chez de nombreux individus de tous âges, actifs ou inactifs, à la suite de traumatismes ou d'efforts trop importants. Les techniques chirurgicales réparatrices couramment employées consistent à attacher le tendon, au moyen d'un fil de suture, à une vis, une cheville ou un piton fixé dans l'os voisin.

Pour cela, il est généralement nécessaire de forer dans l'os un trou et d'y visser une vis ou un piton, ou un trou non taraudé recevant une cheville pouvant se bloquer dans l'os, qui sert de moyen d'ancrage sur lequel on attache ensuite un fil de suture qui est utilisé pour rattacher le tendon qui a été arraché de son support osseux. Suivant d'autres techniques, après avoir foré un trou adapté à recevoir un piton d'ancrage, on fait passer le fil de suture dans le chas du piton d'ancrage, puis on introduit celui-ci dans le trou au moyen d'un appareillage spécial, et enfin on suture le tendon à fixer. Un exemple de vis d'ancrage pour fixation de tissus au moyen d'un fil de suture est décrit dans le brevet US-A-5.443.482.

Selon les cas, les interventions peuvent être effectuées soit en chirurgie à ciel ouvert, soit en chirurgie à ciel fermé au moyen de procédés coelioscopiques ou arthroscopiques. Des techniques chirurgicales utilisant des dispositifs de ce genre sont décrites par exemple par F. A. Barber et al., J. of Arthroscopy and Related Surgery, vol. 11, n° 1, p. 21-28 (1995).

Ces dispositifs connus présentent l'inconvénient d'être très invasifs, non résorbables, et de requérir des techniques de mise en place souvent délicates à mettre en oeuvre; de plus certains produits qui y sont utilisés contiennent des matériaux allergisants, par exemple certains matériaux à mémoire de forme et parfois même cancérigènes, selon certains auteurs. En outre, un grand nombre de vis, de chevilles ou de pitons, et d'accessoires de forage de l'os et de mise en place du moyen d'ancrage, doivent être disponibles dans des dimensions diverses pour pouvoir répondre à toutes les situations.

Dans un autre domaine, la demande WO 95.32669 décrit un dispositif dénommé "pousse-noeud" pour fil de suture, comprenant une tige coulissant dans un tube dont l'extrémité distale est partiellement ouverte, l'ensemble étant manoeuvré par un poussoir solidaire de la tige et d'une plaque d'appui.

La présente invention a pour objet un dispositif d'ancrage osseux nécessitant le forage préalable d'un simple trou à paroi quelconque, sans requérir de taraudage, et permettant la fixation d'organes tels que des tendons et des ligaments, ou la suspension du col de l'utérus, au moyen d'un fil chirurgical ou d'un fil de suture sans qu'il soit nécessaire d'utiliser un moyen du type vis ou piton.

L'invention a également pour objet un matériel ancillaire spécialement adapté à la mise en place du dispositif d'ancrage précité.

Le dispositif d'ancrage conforme à la présente invention est utilisable en combinaison avec un trou foré dans le support osseux, et est constitué par un fil comprenant une partie médiane destinée à être introduite dans le trou foré au préalable, présentant une forme de boucle fermée par un noeud coulant, portant sur la partie en boucle une enveloppe ou gaine tubulaire déformable pouvant coulisser sur le fil dans les limites de la boucle.

Suivant une forme préférentielle de réalisation, le fil utilisé est un fil chirurgical ou un fil de suture, résorbable ou non résorbable, réalisé par exemple en polyester tel que le Ercylène® ou en polyamide tel que le Trynil®.

L'enveloppe ou gaine tubulaire pouvant coulisser sur le fil dans la boucle est d'une longueur totale inférieure ou égale au double de la profondeur du perçage dans l'os et d'un diamètre inférieur ou égal à celui du trou foré dans l'os. Ainsi, la boucle portant la gaine est entièrement introduite dans le trou foré dans l'os. Cette gaine peut avantageusement être réalisée en tous matériaux déformables, et présentant de préférence une certaine élasticité, ayant la propriété d'être implantables, résorbables ou non résorbables (par exemple un fil métallique ou plastique tressé, un tube en polyester ou en polyamide, ou un tube en silicone).

Selon les cas, la gaine peut être constituée par un seul élément ou plusieurs éléments. Lors de la mise en place, les extrémités de la gaine peuvent être en butée contre le noeud coulant, mais ils peuvent aussi être à une certaine distance de celui-ci, la seule condition étant que le fil, lorsque le noeud se resserre, vienne s'appliquer contre la gaine et provoquer sa déformation.

Suivant une forme simple de réalisation, la gaine en forme de cylindre linéaire est enfilée sur le fil, puis la boucle est formée autour de la gaine.

Suivant une variante conforme à l'invention, la paroi de la gaine est percée en son milieu par un orifice par lequel passent les deux brins du fil. Ainsi, le fil pénètre dans la gaine par son milieu, en sort une première fois par une extrémité, forme une boucle et rentre à nouveau par l'autre extrémité de la gaine et ressort par l'orifice pratiqué dans le milieu de la gaine.

Suivant une autre forme de réalisation de l'invention, la gaine se présente sous la forme d'un anneau torique présentant au moins un orifice traversant sa paroi pour le passage des deux brins du fil. Dans cette variante, le fil pénètre dans la gaine par l'orifice, forme une boucle en suivant l'intérieur de la gaine annulaire et ressort par le même orifice.

La gaine est de préférence renforcée dans la zone où pénètre le fil, c'est-à-dire sur la périphérie de chacune de ses extrémités, ou autour de l'orifice pratiqué dans sa paroi, le cas échéant. Ce renforcement peut être obtenu par exemple en prévoyant une surépaisseur de matière ou en fixant par collage une bande présentant une résistance plus grande.

L'ancillaire de mise en place du dispositif d'ancrage conforme à la présente invention comprend un manche comportant à une extrémité une pointe susceptible de porter la boucle du fil et d'être introduite dans le trou foré dans l'os, et à l'autre extrémité une poignée de manipulation par l'utilisateur, ainsi que au moins un support porte-fil solidaire du manche et portant les extrémités du fil.

Suivant une forme préférentielle de réalisation, la poignée peut coulisser longitudinalement par rapport au manche sur une distance limitée par au moins une butée, et le manche ou la poignée comporte des moyens pour bloquer leur position relative tant qu'une force prédéterminée de traction n'est pas appliquée sur la poignée ou sur le manche.

Suivant une autre caractéristique de la présente invention, l'ancillaire comprend deux supports porte-fil, chacun étant constitué par une plaquette disposée sur le manche, les deux plaquettes se trouvant de part et d'autre de l'axe du manche, et étant susceptibles de coulisser sur le manche suivant une distance différente l'une de l'autre. Sur la plaquette pouvant coulisser sur la plus grande distance est fixée l'extrémité du brin du fil portant le noeud coulant, tandis que l'extrémité de l'autre brin du fil, qui ne porte pas le noeud coulant, est rattachée à la plaquette fixe ou de plus faible coulissement.

L'utilisation de l'ancillaire pour la mise en place du dispositif d'ancrage de l'invention se fait facilement en introduisant la pointe de l'ancillaire, portant la boucle et sa gaine, dans le trou préalablement foré dans l'os, jusqu'à ce que la boucle soit totalement engagées dans le trou. Après avoir retiré la pointe hors du trou, tout en y laissant le fil et la gaine, il suffit alors d'exercer une traction sur le brin du fil ne portant pas le noeud coulant, tout en freinant légèrement l'autre brin, pour provoquer le resserrement de la boucle à l'intérieur du trou dans l'os et la déformation de la gaine sur la boucle jusqu'à prendre la forme d'une boule dont le diamètre est supérieur à celui de la gaine tubulaire avant déformation. La traction étant prolongée jusqu'au blocage, la gaine déformée se trouve alors en compression contre les parois internes de la cavité.

Comme indiqué ci-dessus, le dispositif et l'ancillaire de pose suivant la présente invention sont tout particulièrement destinés à la chirurgie réparatrice de ligaments et tendons. L'invention peut aussi être utilisée en gynécologie, par exemple pour fixer l'utérus sur le sacrum par l'intermédiaire d'un ligament.

Le dispositif de l'invention convient encore à la chirurgie carcinologique, et dans cette application on utilise un fil qui peut contenir une substance radioactive telle que de l'iridium. Le fil est placé dans la tumeur, et en utilisant de préférence un fil non résorbable, ce dernier peut servir de repère pour l'ablation secondaire de la tumeur.

Les essais réalisés avec le dispositif d'ancrage suivant la présente invention, effectués dans des conditions expérimentales, ont permis de mettre en évidence d'excellentes propriétés de fixation et de résistance à la traction, comparable à celles des meilleurs dispositifs connus de la technique. Ainsi, en utilisant un fil de suture en polyglatine tressé résorbable de 0,4 mm de section portant, au niveau de la boucle, une gaine en polyester tressé non résorbable de 0,6 mm de section, introduit dans une série de trous de 1,6 mm de diamètre et 18 mm de profondeur forés dans un fémur de mouton frais provenant d'un abattoir, la résistance à l'arrachement dans l'axe du trou est d'environ 12 kg. Dans tous les cas on observe une rupture du fil de suture sans extraction de la gaine hors du trou.

Les caractéristiques et avantages de la présente invention apparaîtront plus clairement dans les exemples suivants relatifs à des formes préférentielles de réalisation, en référence aux dessins annexés, qui représentent:
Figure 1 : une vue schématique d'un fil de fixation conforme à la présente invention.
Figure 2 : une vue en perspective d'un matériel ancillaire simplifié pour la mise en place du dispositif d'ancrage de la Figure 1.
Figure 3 : une vue en perspective d'un matériel ancillaire perfectionné par rapport à celui de la Figure 2, permettant une mise en place semi-automatique d'un dispositif d'ancrage selon la Figure 1.
Figures 4a et 4b : le détail du manche du matériel de la Figure 3 et de la poignée qui s'y insère.
Figure 5 : une vue d'un élément support pouvant recevoir l'élément de la Figure 6, similaire à celui de la Figure 3, pour la mise en place automatique.
Figure 6 : une vue de l'élément se combinant à celui de la Figure 5 pour la mise en place automatique du dispositif d'ancrage.
Figure 7 : une vue de détail montrant la position du fil et de la gaine du dispositif d'ancrage sur la pointe d'un matériel ancillaire tel que représenté sur les Figures ci-dessus.

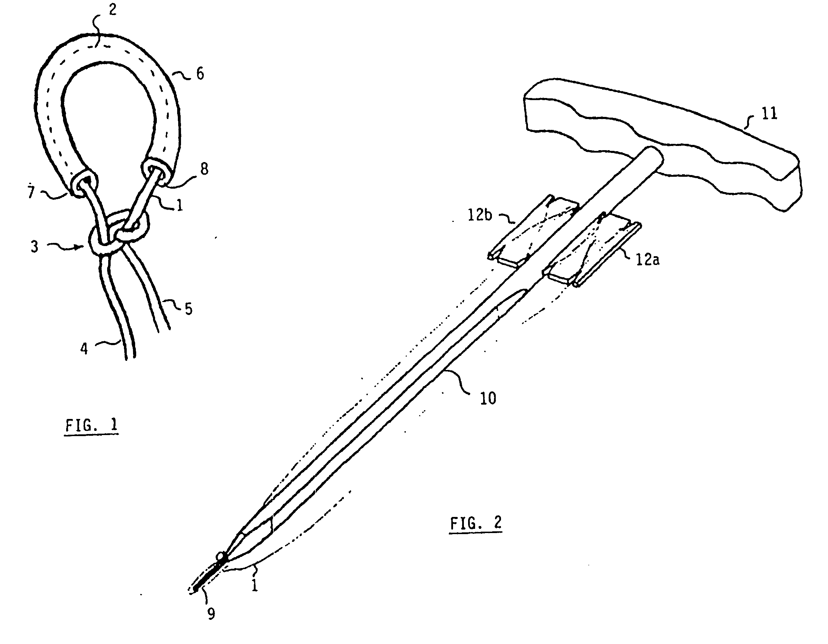

Comme le montre la Figure 1, le dispositif d'ancrage de la présente invention comprend un fil de suture (1) formant dans sa partie médiane une boucle fermée (2) par un noeud coulant (3) susceptible d'être serré par simple traction sur l'un des deux brins (4, 5) du fil (1).

Dans la boucle (2), le fil (1) est enveloppé par une gaine (6), déformable et compressible, susceptible de glisser sur le fil. La traction sur le brin (4) du fil provoque une diminution de la longueur de la boucle (2) jusqu'à ce que sa longueur devienne égale à celle de la gaine (6). En continuant la traction sur le brin (4) du fil (1), on provoque d'une part une compression de la gaine dont la surface forme des ondulations en raison de la compressibilité de la matière qui la constitue, et d'autre part le serrage du noeud (3). Les bords de la gaine, à chacune de ses extrémités (7) et (8), sont renforcés pour éviter qu'ils ne soient entaillés par le fil de suture lorsque celui-ci est serré. Ce renforcement est obtenu ici par une simple surépaisseur de matière.

La mise en place du dispositif s'effectue au moyen d'un matériel ancillaire tel que décrit ci-après. La méthode de mise en place consiste à introduire la boucle (2) portant la gaine (6) dans le trou foré dans l'os, puis à serrer en exerçant une traction sur le brin (4) du fil (1). Il est préférable d'introduire la totalité de la boucle dans le trou ainsi que le noeud coulant (3).

Lorsque l'on serre le noeud coulant (3) en tirant sur le brin (4) du fil, on provoque le rétrécissement de la boucle et la compression de la gaine flexible (6) à l'intérieur du trou. Puis en accentuant le serrage par traction sur le brin (4) du fil (1) on déforme la gaine (6) jusqu'à ce qu'elle prenne la forme d'une boule. Cette boule ne pourra ressortir par le trou par lequel on l'a introduite dans l'os car son diamètre est devenu nettement supérieur à celui du trou foré. De plus cette boule prend appui sur la face interne de l'os cortical ou dans l'os spongieux s'il est suffisamment dur.

Il suffit donc de choisir un fil de suture portant une gaine ayant une longueur et un diamètre permettant, par serrage du noeud coulant (3), la formation d'une boule qui prendra appui sur la face interne dure d'un os et dont le diamètre sera suffisamment important pour résister aux efforts qui lui sont imposés.

La pose du dispositif d'ancrage décrit ci-dessus s'effectue efficacement au moyen d'un matériel ancillaire conforme à l'invention, représenté sur la Figure 2.

Sur cette Figure est représenté un matériel ancillaire simple facilitant la mise en place du dispositif d'ancrage dans un trou préalablement foré dans un os.

Cet ancillaire comprend une pointe (9) destinée à supporter la boucle du fil de suture (1) pour assurer sa mise en place dans le trou foré dans l'os (non représenté). Cette pointe (9) est fixée sur un manche (10) solidaire d'une poignée (11). Sur le manche (10) à proximité de la poignée (11) sont disposées deux plaquettes (12a) et (12b) sur lesquelles on place les deux extrémités du fil de suture (1) afin de faciliter leur manipulation par l'utilisateur.

Après forage du trou dans l'os au moyen d'un appareil usuel, on introduit la pointe (9) portant le fil (1) formé en boucle portant sa gaine (6), dans le trou, puis on défait les brins (4) et (5) du fil (1) fixés sur les plaquettes (12a) et (12b). On retire l'ancillaire du trou en agissant sur la poignée (11), puis on tire sur le brin (4) du fil (1) de manière à faire glisser le noeud coulant (3) et à déformer la gaine (6) pour qu'elle forme une boule.

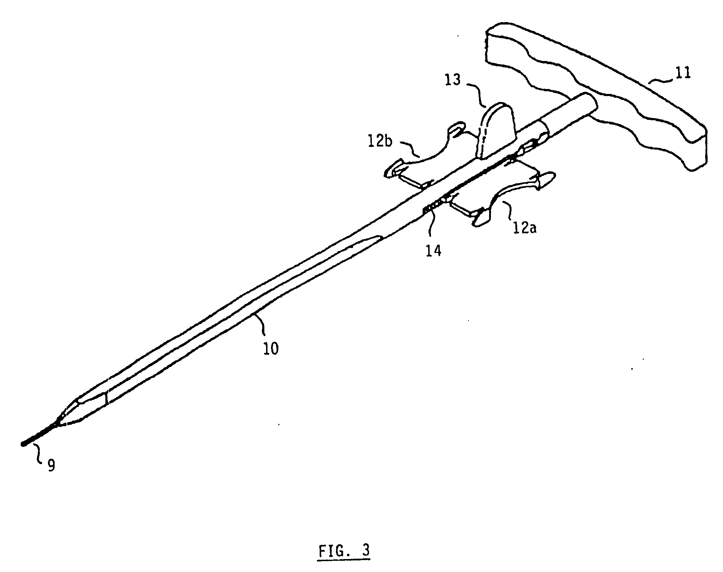

L'ancillaire représenté sur la Figure 3 comporte une pointe (9), un manche (10), une poignée (11) et des plaquettes (12a) et (12b) identiques à ceux de la Figure 2, mais la poignée et les plaquettes peuvent se déplacer axialement par rapport au manche.

Une goupille (13) amovible permet de bloquer les deux plaquettes porte-fil (12a) et (12b) tandis que la poignée est maintenue en place sur la manche par simple encliquetage. Le manche (10) est fendu par une rainure (14) qui permet le déplacement des plaquettes lorsque la goupille (13) est enlevée. Cette rainure (14) est plus longue du côté de la plaquette (12b) que du côté de la plaquette (12a) de manière à limiter le mouvement des plaquettes de manière différenciée, comme indiqué ci-après, le mouvement de translation de la plaquette (12b) pouvant s'effectuer sur une distance plus longue que celui de la plaquette (12a). Ainsi, le brin du fil formant le noeud est fixé à la plaquette dont le mouvement est le plus important.

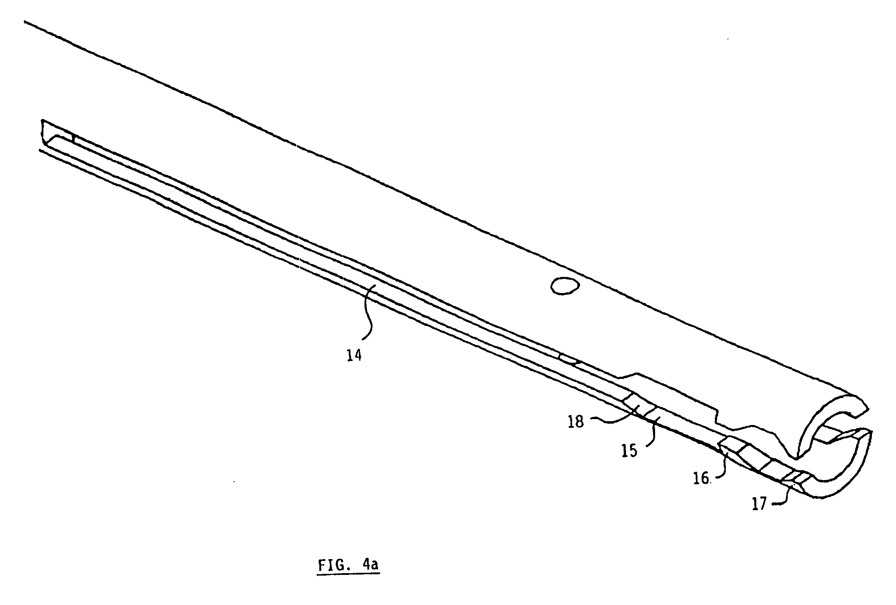

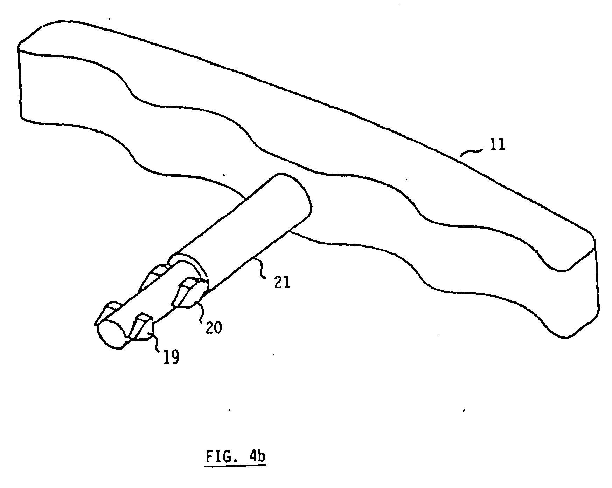

Comme le montre la Figure 4a, la rainure (14) est élargie dans la partie (15) recevant la poignée. Cette partie élargie (15) de la rainure comporte une butée (16), et elle est limitée par les deux épaulements (17) et (18) formés sur chaque moitié du manche. Cette butée et ces épaulements sont destinés à coopérer avec deux ergots (19) et (20) prévus sur l'axe (21) de la poignée (11).

Lorsque la poignée est en place, son axe (21) est introduit dans le manche (10) jusqu'à ce que l'ergot (19) vienne en appui contre l'épaulement (18), l'ergot (20) étant alors en appui contre la partie inclinée de la butée (16).

La tête de l'ergot (19) est de forme triangulaire, la pointe étant dirigée vers le fond de la rainure (14) tandis que sa base est dirigée vers la poignée (11). Par contre, l'ergot (20) a une tête à forme hexagonale. De la sorte, lorsque l'on tire avec une force suffisante sur la poignée (11) préalablement mise en place sur le manche (10), l'ergot (20), en raison de sa forme, peut passer sur la butée (17) en écartant les deux branches du manche (10) de part et d'autre de la rainure (14), le déplacement étant limité par l'ergot (19) dont la base vient contre la butée (16). Dans cette position, l'ergot (20) a dépassé l'épaulement (17).

Ce mouvement de la poignée (11) par rapport au manche (10) ne peut s'effectuer qu'en exerçant une force de traction prédéterminée sur la poignée, de manière à surmonter la résistance de la butée (17) contre l'un des pans hexagonaux de l'ergot (20) et à forcer l'écartement des deux branches du manche de part et d'autre de la rainure (14). Ainsi, une faible traction sur la poignée (11) entraîne le manche (10) avec la poignée, tandis qu'une traction plus forte désolidarise la poignée du manche.

La mise en place du dispositif d'ancrage au moyen du matériel ancillaire de la Figure 3 s'effectue de manière semi-automatique comme décrit ci-après.

Le dispositif d'ancrage est tout d'abord introduit dans un trou foré au préalable dans l'os. A cet effet, la boucle fermée portant la gaine flexible est placée sur la pointe (9) et les extrémités du fil de suture sont fixées sur les plaquettes porte-fil (12a) et (12b) comme dans l'exemple précédent, les deux brins du fil étant tendus entre la pointe et chaque plaquette.

La pointe portant le fil est introduite dans le trou foré dans l'os et la goupille (13) est enlevée afin de libérer les deux plaquettes qui peuvent coulisser sur le manche (10). L'utilisateur exerce une légère traction sur la poignée, qui provoque le retrait de la pointe du trou, la poignée et le manche restant solidaires. Dans le même temps la boucle, sa gaine et le noeud, restent dans le trou, en raison des forces de frottement. Puis étant donné que les plaquettes peuvent coulisser sur le manche (10), la plaquette (12a) va venir en appui contre le fond de la rainure (14) et va tendre le brin (4). Ceci va provoquer le serrage du noeud (3) et la déformation de la gaine (6) jusqu'à ce qu'elle forme une boule. Si l'utilisateur poursuit sa traction sur la poignée, au-delà d'une valeur prédéterminée, la force de traction est supérieure à la résistance opposée par la butée (17) sur l'ergot hexagonal (20) et la poignée coulisse alors par rapport au manche jusqu'à ce que l'ergot triangulaire (19) vienne contre la butée (16). Ceci permet de régler la force à exercer sur le brin (4) pour assurer l'ancrage de la gaine (6) qui s'est transformée en boule, et le fait que l'ergot (15) vienne en butée contre (16) évite au manche (10) de tomber.

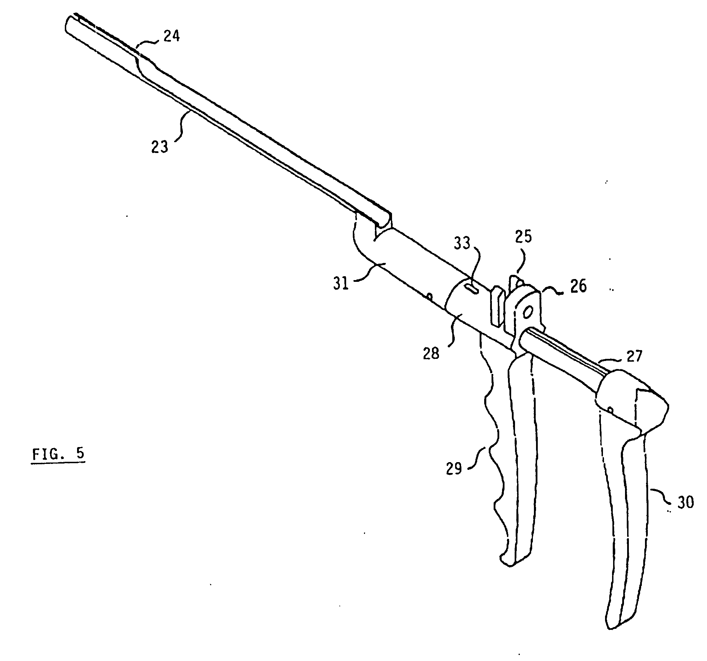

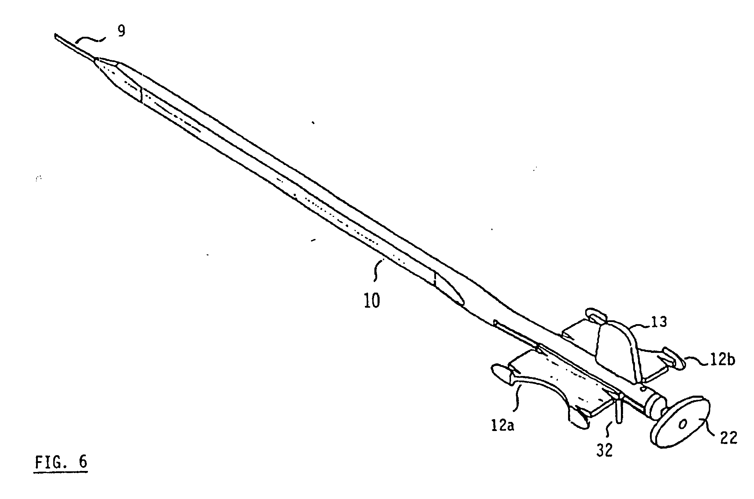

Le matériel ancillaire dont les deux éléments constitutifs sont représentés sur les Figures 5 et 6 est destiné à une mise en place automatique du dispositif d'ancrage de l'invention, l'utilisateur pouvant dans ce cas manipuler l'ancillaire d'une seule main.

L'élément de la Figure 5 est un support recevant celui de la Figure 6. Ce dernier est similaire au matériel ancillaire de la Figure 3, la poignée (11) étant remplacée par un poussoir (22).

L'élément support de la Figure 5 comprend un support hémicylindrique (23) destiné à recevoir le manche (10) de l'élément de la Figure 6. Pour assurer un bon maintien du manche, ce support est refermé dans sa partie distale (24). Ainsi la mise en place de l'autre élément se fait en introduisant l'extrémité du manche (10) portant la pointe (9) dans la partie ouverte du support (23) et en la faisant glisser dans la partie fermée (24) jusqu'à ce que le poussoir (22) vienne entre les deux butées (25) et (26) solidaires du support.

La partie (28) du support portant les butées (25) et (26) peut coulisser sur l'axe (27), le mouvement de coulissement étant effectué en agissant sur la poignée (29) solidaire du support (28) et sur la poignée (30) solidaire de l'axe (27) (les deux poignées sont maintenues écartées par un ressort non représenté). L'extrémité de l'axe (27) est solidaire de la partie (31) du support qui porte le tube ouvert (23). Lorsque la goupille (13) est en place, son axe (32) s'engage dans le trou (33) prévu dans le support (28) et l'axe (27) et ainsi les deux poignées sont bloquées l'une par rapport à l'autre.

L'utilisateur saisit l'ancillaire, comprenant les deux éléments des Figures 5 et 6 assemblés, par les deux poignées (29) et (30) d'une seule main, il engage la pointe (9) portant la boucle et la gaine dans le trou préalablement foré dans l'os, puis il enlève la goupille (13) libérant ainsi les poignées (29) et (30). En agissant sur les poignées de manière à les rapprocher, il provoque un mouvement de retrait du manche (10) qui coulisse dans le support hémicylindrique (23) provoquant le coulissement de la plaquette (12a) par rapport au manche (10) jusqu'à venir en butée contre le fond de la rainure (14), puis le brins (4) se tend et provoque le serrage du noeud (3) et la transformation de la gaine (6) en boule. L'utilisateur doit continuer à serrer les poignées jusqu'à ce que le poussoir (22) se casse au niveau de son rétrécissement. Ceci indique à l'utilisateur qu'il a exercé la force nécessaire à la mise en place du système d'ancrage.

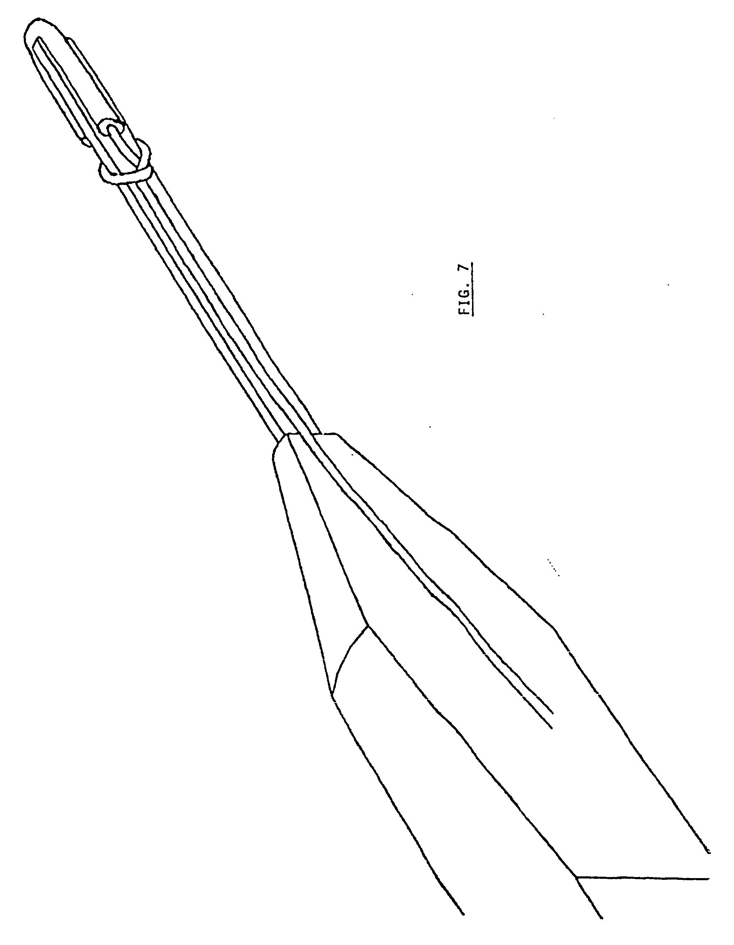

La Figure 7 montre explicitement la position du fil de suture (1) en boucle fermée et portant la gaine flexible (3) mis en place sur la pointe (9) à l'extrémité du manche (10) de l'ancillaire de pose.

## Revendications

1. Dispositif chirurgical d'ancrage osseux utilisable en chirurgie orthopédique, traumatologique, gynécologique et carcinologique, en combinaison avec un trou foré au préalable dans le support osseux, comprenant un fil (1) en forme de boucle (2) fermée par un noeud coulant (3), **caractérisé en ce que** la partie médiane du fil (1), destinée à être introduite dans le trou, porte une gaine tubulaire déformable (6) pouvant coulisser sur le fil (1) dans les limites de la boucle (2), et transformable en boule par traction exercée sur au moins un brin (4) du fil (1).

2. Dispositif selon la revendications 1, **caractérisé en ce que** la longueur de la gaine (6) est inférieure ou égale au double de la profondeur du trou foré dans le support osseux.

3. Dispositif selon l'une quelconque des revendications précédentes, **caractérisé en ce que** le diamètre de la gaine (6) est inférieur ou égal au diamètre du trou.

4. Dispositif selon l'une quelconque des revendications précédentes, **caractérisé en ce que** la gaine (6) est constituée par un seul élément ou plusieurs éléments.

5. Dispositif selon la revendication 4, **caractérisé en ce que** la gaine (6) est constituée par un seul élément cylindrique linéaire ouvert à ses deux extrémités (7, 8).

6. Dispositif selon la revendication 5, **caractérisé en ce que** la gaine comporte au moins un orifice traversant sa paroi pour le passage des deux brins du fil.

7. Dispositif selon la revendication 4, **caractérisé en ce que** la gaine (6) est constituée par un anneau torique présentant au moins un orifice traversant sa paroi pour le passage des deux brins du fil.

8. Ancillaire de mise en place du dispositif d'ancrage suivant la revendication 1, comprenant un manche (10) comportant à une extrémité une poignée (11) de manipulation par l'utilisateur, et au moins deux supports porte-fil (12a, 12b) solidaires du manche (10) et portant les extrémités du fil, **caractérisé en ce que** le manche (10) comprend à l'autre extrémité une pointe (9) susceptible de porter la boucle (2) du fil (1) et d'être introduite dans le trou foré dans l'os.

9. Ancillaire selon la revendication 8, **caractérisé en ce que** la poignée (11) peut coulisser longitudinalement par rapport au manche (10) sur une distance limitée par au moins une butée (16), et le manche ou la poignée comporte des moyens (16, 19) pour bloquer leur position relative tant qu'une force prédéterminée de traction n'est pas appliquée sur la poignée (11) ou sur le manche (10).

10. Ancillaire selon l'une quelconque des revendications 8 et 9, **caractérisé en ce que** chaque support porte-fil (12a, 12b) est constitué par une plaquette disposée sur le manche (10), les deux plaquettes se trouvant de part et d'autre de l'axe du manche, et étant susceptibles de coulisser sur le manche suivant une distance différente l'une de l'autre.

11. Ancillaire selon la revendication 10, **caractérisé en ce que** l'extrémité du brin du fil portant le noeud coulant est fixée sur la plaquette pouvant coulisser sur la plus grande distance, tandis que l'extrémité de l'autre brin du fil, qui ne porte pas le noeud coulant, est rattachée à la plaquette fixe ou de plus faible coulissement.

## Patentansprüche

1. Chirurgische Vorrichtung, die in der orthopädischen, traumatologischen, gynäkologischen und karzinologischen Chirurgie zur Verankerung von Knochen in einem im Voraus im Knochenträger gebohrten Loch einsetzbar ist, umfassend einen Faden (1) in Form einer durch eine Schlaufe (3) geschlossenen Schlinge (2), **dadurch gekennzeichnet, dass** der Mittelteil des Fadens (1), der in das Loch einzubringen ist, eine verformbare, röhrenförmige Hülse (6) trägt, die innerhalb der Schlinge (2) über den Faden (1) gleiten kann und durch Zugeinwirkung an zumindest einem Teilfaden (4) des Fadens (1) kugelförmig verformbar ist.

2. Vorrichtung nach Anspruch 1, **dadurch gekennzeichnet, dass** die Länge der Hülse (6) kleiner als die oder gleich der doppelte(n) Tiefe des im Knochenträger gebohrten Lochs ist.

3. Vorrichtung nach einem der vorangegangenen Ansprüche, **dadurch gekennzeichnet, dass** der Durchmesser der Hülse (6) kleiner als der oder gleich dem Durchmesser des Lochs ist.

4. Vorrichtung nach einem der vorangegangenen Ansprüche, **dadurch gekennzeichnet, dass** die Hülse (6) aus einem oder mehreren Elementen besteht.

5. Vorrichtung nach Anspruch 4, **dadurch gekennzeichnet, dass** die Hülse (6) aus einem einzigen an seinen beiden Enden offenen (8, 9), geraden zylindrischen Element besteht.

6. Vorrichtung nach Anspruch 5, **dadurch gekennzeichnet, dass** die Hülse zumindest ein Durchgangsloch in ihrer Wand zum Durchführen der zwei Teilfäden des Fadens umfasst.

7. Vorrichtung nach Anspruch 4, **dadurch gekennzeichnet, dass** die Hülse (6) aus einem torischen Ring besteht, der zumindest ein Durchgangsloch in seiner Wand zum Durchführen der zwei Fadenenden aufweist.

8. Hilfsvorrichtung zur Anbringung der Verankerungsvorrichtung nach Anspruch 1, umfassend einen Stiel (10) mit einem Griff (11) an einem seiner Enden zur Handhabung durch den Benutzer und zumindest zwei Fadenträger (12a, 12b), die am Stiel (10) angebracht sind und die Enden des Fadens tragen, **dadurch gekennzeichnet, dass** der Stiel (10) am anderen Ende eine Spitze (9) umfasst, die zum Tragen der Schlinge (2) des Fadens (1) vorgesehen und in das im Knochen gebohrte Loch eingeführt werden kann.

9. Hilfsvorrichtung nach Anspruch 8, **dadurch gekennzeichnet, dass** der Griff (11) entlang dem Stiel (10) über einen durch zumindest einen Anschlag (16) begrenzten Abschnitt gleiten kann und der Stiel oder der Griff Mittel (16, 19) umfassen, um ihre relative Position zu fixieren, wenn eine vorbestimmte Zugkraft auf den Griff (11) oder den Stiel (10) nicht ausgeübt wird.

10. Hilfsvorrichtung nach einem der Ansprüche 8 und 9, **dadurch gekennzeichnet, dass** jeder Fadenträger (12a, 12b) aus einer am Stiel (10) angebrachten Platte besteht, wobei sich die zwei Platten auf den zwei gegenüberliegenden Seiten der Stielachse befinden und in der Lage sind, unabhängig voneinander über eine unterschiedliche Distanz entlang dem Stiel zu gleiten.

11. Hilfsvorrichtung nach Anspruch 10, **dadurch gekennzeichnet, dass** das Ende des die Schlaufe tragenden Teilfadens des Fadens auf der Platte fixiert ist, die über die größere Distanz gleiten kann, während das Ende des anderen Teilfadens des Fadens, der nicht die Schlaufe trägt, an der Platte angebracht ist, die stationär ist oder über eine kürzere Distanz gleitet.

## Claims

1. Surgical device for anchorage in bone that can be used in orthopedic, traumatological, gynecological and carcinological surgery, in combination with a hole bored beforehand in the bony support, comprising a thread (1) in the form of a loop (2) closed by a running knot (3), **characterized in** the middle part of the thread (1), which is intended to be introduced into the hole, carries a deformable tubular sleeve (6) which can slide along the thread (1) within the limits of the loop (2), and can be made into a loop by tension exerted on at least one strand (4) of the thread (1).

2. Device according to Claim 1, **characterized in that** the length of the sleeve (6) is less than or equal to twice the depth of the hole bored in the bony support.

3. Device according to any of the preceding claims, **characterized in that** the diameter of the sleeve (6) is less than or equal to the diameter of the hole.

4. Device according to any of the preceding claims, **characterized in that** the sleeve (6) is made of a single element or of several elements.

5. Device according to Claim 4, **characterized in that** the sleeve (6) is made of a single linear cylindrical element open at both ends (7, 8).

6. Device according to Claim 5, **characterized in that** the sleeve comprises at least one orifice passing through its wall for the passage of the two strands of thread or wire.

7. Device according to Claim 4, **characterized in that** the sleeve (6) is made of a toric ring having at least one orifice passing through its wall for the passage of the two strands of thread or wire.

8. Ancillary for inserting the anchoring Device according to Claim 1, comprising a shaft (10) having a handle (11) at one end for manipulation by the user, and at least one thread-carrying support (12a, 12b) secured to the shaft (10) and carrying the ends of the thread or wire, **characterized in that** the shaft (10) comprises at the other end a spike (9) that can hold the loop (2) of thread (1) and be introduced into the hole bored in the bone.

9. Ancillary according to Claim 8, **characterized in that** the handle (11) can slide longitudinally with respect to the shaft (10) over a distance which is limited by at least one stop (16), and the shaft or the handle comprises means (16, 19) for locking their relative position until a predetermined tensile force is applied to the handle (11) or to the shaft (10).

10. Ancillary according to either one of claims 8 and 9, **characterized in that** each thread-carrying support (12a, 12b) consists of a small plate arranged on the shaft (10), the two small plates being situated one on either side of the axis of the shaft, and capable of sliding along the shaft over distances which differ from one another.

11. Ancillary according to claim 10, **characterized in that** the end of the strand of thread carrying the running knot is fixed to the small plate that can slide over the longest distance, while the end of the other strand of thread or wire, which does not carry the running knot, is attached to the small plate which is stationary or slides over a shorter distance.
